# EUROPEAN PATENT SPECIFICATION

(11) **EP 0 306 083 B1**
(45) Date of publication and mention of the grant of the patent: **31.03.1993**
(21) Application number: 88201813.8
(22) Date of filing: 25.08.1988
(51) Int. Cl.: C04B 35/76, C22C 29/12, A61L 27/00

(54) **Method of manufacturing a ceramic body filled with metal particles**
Verfahren zum Herstellen einer mit Metallteilchen gefüllten keramischen Körpers
Procédé pour la réalisation d'un corps céramique rempli de particules métalliques

(30) Priority: 02.09.1987 NL 8702058
(43) Date of publication of application: 08.03.1989
(73) Proprietor: Philips Electronics N.V., 5621 BA Eindhoven (NL)
(72) Inventor: de With, Gijsbertus, NL-5656 AA Eindhoven (NL)
(74) Representative: Pennings, Johannes

(56) References cited:
- EP-A- 0 104 640
- GB-A- 1 550 330
- US-A- 3 321 285
- JOURNAL OF THE AMERICAN CERAMIC SOCIETY, vol. 60, no. 9/10, September-October 1977, pages 390-396; J.G. ZWISSLER et al.: "Strength and toughness of a ceramic reinforced with metal wires"

## Description

The invention relates to a method of manufacturing a ceramic body filled with metal particles and to a ceramic body manufactured by means of a method according to the invention.

A method of manufacturing a ceramic material in which a mixture in powder form of metal particles and ceramic particles is cold-compressed and sintered is described in Japanese patent application (Kokai) JP 59-107058, see Patent Abstracts of Japan, 8 (222), page 121 C 246 (1984). The metal particles, preferably metal fibres, have for their object to increase the heat resistance of the ceramic material.

It is known per se to fill a ceramic material with a finely divided material, for example, to influence electrical or mechanical properties. The filler may also be used inter alia in the form of powder, grains, flakes or fibres. For most applications it is of importance for the filler to be readily mixed with the basic material. When the filler is used for the mechanical reinforcement of the composite material it is necessary that a bond is formed between the particles and the basic material to prevent that the particles become located as it were in cavities in the basic material.

In particular when metal particles are used as fillers the problem occurs that it is difficult to obtain a good mixing without damaging the metal particles. For example, the breaking and shortening of fibres leads to a decrease of the reinforcing effect. A conventional manner of mixing, for example in a ball mill, is therefore undesired because due to the long residence damage occurs and also because there is a fibre agglomerating effect which is disadvantageous for the homogeneity. A material which has not been thoroughly mixed leads to inhomogeneities and density differences and ultimately to deformation of the final product.

A conventional method of mixing a slurry containing ceramic particles with metal filaments in a ball mill is described in US patent specification 3,321,285.

It is the object of the invention to provide a method with which a good mixing of metal particles and ceramic particles is obtained, in which the metal particles are not damaged or are damaged only to an insignificant extent. Another object of the invention is to provide a method with which a homogeneous ceramic body can be manufactured which shows accurately the desired shape and dimensions.

According to the invention this object is achieved by means of a method in which metal particles and ceramic particles are mixed wet in a grinder, in which large shearing forces occur, after which the mixed mass is dried and ground to a coarse powder, the powder is then compressed to the desired shape in a jig, the thus formed intermediate product is then densified by means of isostatic compression and is then sintered at elevated temperature and pressure.

The steps of wet mixing and grinding to a coarse powder are carried out in an as short period of time as possible, for example, by using a grinder which has knives which rotate at a high speed, for example, in a hammer mill. The good homogeneity is obtained in that the particles of the coarse powder which is used to fill the jig each consist of a mixture of the desired composition.

In order to obtain a good homogeneous filling of the jig, it is to be preferred that the coarse powder is produced into the jig in the form of thin layers, each layer being smoothed before the next layer is provided.

The coarse powder in the jig is preferably compressed to the desired shape at a pressure of 10⁷ to 5.10⁷ Pa.

The densification step by means of isostatic compression is preferably carried out at a pressure of 2.10⁸ to 5.10⁸ Pa.

If it is desirable to prevent oxidation or corrosion of the metal particles, it may be advantageous to perform the sintering step in an inert atmosphere. The sintering step is preferably carried out at a temperature from 900 to 1000°C and a pressure of 3.10⁷ to 10⁸ Pa.

Although during the densification step and the sintering densification takes place and hence a reduction of the dimensions of the ceramic body, said densification is very much reproducible and predictable due to the high degree of homogeneity which is obtained in the method according to the invention so that no problems occur in designing a product.

In order to obtain a mechanically strong ceramic body it is to be preferred that metal fibres are used as metal particles having a length smaller than 2 mm and a diameter from 5 to 100 µm. Larger particles cannot easily be mixed homogeneously with the basic material.

The greatest reinforcement is obtained when the fibres are tensile loaded and the basic material experiences a compression. This can be achieved when the metal particles used are fibres having a higher coefficient of linear expansion than that of the ceramic basic material. Because metal fibres are plastically deformable it is not very objectionable to use metals the coefficient of linear expansion of which is slightly lower than that of the matrix material.

In order to improve the bonding between the metal fibres and the matrix material, or just to prevent undesired reactions between the metal fibres and the matrix material, it is efficacious to use fibres having a core of a metal with suitable mechanical properties, which core is covered with a bonding or protecting envelope.

In a preferred embodiment of the ceramic body obtained by means of the method according to the invention the ceramic material is hydroxy apatite. In that case a particularly good result is obtained if the metal particles consist of fibres of an iron-chromium alloy. The said alloy may additionally comprise other elements, for example aluminium, nickel and molybdenum. Due to its biocompatible properties, hydroxy apatite is suitable for use as an artificial bonde.

The invention will now be described in greater detail with reference to specific examples.

### Example 1.

According to this example, metal fibres are used having an average length of approximately 1 mm and an average diameter of approximately 15 µm. The fibres consist of 23 % by weight of Cr, 14 % by weight of Fe, 62 % by weight of Ni, balance Al, Si and C. The coefficient of linear expansion of the metal fibres used in this example and the following examples is approximately 12.10⁻⁶/°C.

The metal fibres are washed in water and then dried and sieved through a sieve having meshes of 1.5 mm. A mixture is made of 10 % by vol. of metal fibres and 90 % by vol. of Ca₅(PO₄)₃.OH (hydroxy apatite).

10 0 g of the materials to be mixed and 140 g of water are intimately mixed in a coffee mill for 5 seconds. The mixture is then scraped from the walls of the coffee mill and mixed again for another 5 seconds. The times chosen are short so as to prevent fracture of the metal fibres as much as possible.

The resulting mass is dried. A cake is obtained which is broken and is reduced to a coarse powder by means of a sieve having meshes of 1.5 mm. The powder consists of grains which comprise both metal fibres and hydroxy apatite particles.

The powder is provided in a synthetic resin jig, for example of polymethylmethacrylate, in the form of layers of 5 mm thickness which are always smoothed before the next layer is provided, by which method formation of cavities should be prevented. The powder is then compressed to the desired shape while using a pressure of 10⁷ to 5.10⁷ Pa depending on the dimensions of the product.

The intermediate product is then densified by means of isostatic compression in a vessel in which the product is carefully packaged to avoid direct contact with the oil. The pressure is 5.10⁸ Pa.

The product is finally hot pressed in a nitrogen atmosphere at a pressure of 7.10⁷ Pa. The heating up rate is 200°C/h and the product is kept at the peak temperature of 1000°C for 15 minutes. This step in which sintering occurs is carried out in an as short as possible period of time to prevent damage of the metal fibres.

A number of properties have been measured at the resulting product. The modulus of elasticity E is measured via the longitudinal speed of sound in which on theoretical grounds it is assumed that the Poisson's constant is equal to 0.275. K denotes the tear resistance which is measured in a three-point bending test, at a one-sided notched test piece. The Knoop hardness H is measured by means of a diamond imprint at a load of 200 g for 15 seconds. σₘₐₓ is the maximum bending strength measured in a three-point bending test. The density ρ is indicated as a fraction of the theoretical density, that is to say the density with maximum packing of the composing parts. The results are as follows:
modulus of elasticity E = 121 GPa,
resistance to tear K = 4.4 MPa.m^{½},
Knoop hardness H = 4.0 GPa,
density ρ = 98 %.

For comparison these properties have also been measured at unfilled hydroxy apatite which has been prepared in the same manner with the only difference that the warming up rate during sintering is 60°C/h in connection with the high coefficient of expansion (14 to 15.10⁻⁶/°C) and the poor thermal conductivity of unfilled hydroxy apatite:
modulus of elasticity E = 85 GPa,
resistance to tear K = 1.3 MPa.m^{½},
Knoop hardness H = 5.4 GPa,
tensile strength σₘₐₓ = 77 MPa,
density ρ = 93 %.

### Example 2.

According to this example metal fibres are used having an average length of approximately 1 mm and an average diameter of approximately 8 µm. The fibres consist of 22 % by weight of Cr, 18 % by weight of Fe, 48 % by weight of Ni, 9 % by weight of Mo, balance C, Co and W.

The method used is the same as in the preceding example in which the pressure during the isostatic compression is 4.10⁸ Pa and the pressure during sintering is 5.10⁷ Pa.

Products are manufactured with 10, 20 and 30 % by volume of metal fibres. The properties are measured as indicated in the preceding example. The results are recorded in table 1.

**Table 1**

| Vol. fraction metal fibres | E (GPa) | K (MPa.m^{½}) | H (GPa) | ρ (%) |
|---|---|---|---|---|
| 10 | 120 | 4.3 | 4.5 | 96 |
| 20 | 107 | 6.0 | 4.5 | 94 |
| 30 | 141 | 6.1 | 4.2 | > 99 |

The definitions of the measured properties and the comparative values of unfilled hydroxy apatite are recorded in example 1.

### Example 3.

According to this example metal fibres are used having an average length of approximately 1 mm and an average diameter of approximately 22 µm. The fibres consist of 16 % by weight of Cr, 97 % by weight of Fe, 5 % by weight of Al and traces of C and Si.

The method used is the same as in the preceding examples in which the pressure during the isostatic compression is 4.10⁸ and the pressure during sintering is 7.10⁷ Pa.

Products are manufactured with 10, 20 and 30 % by volume of metal fibres. The properties are measured as indicated in example 1. The results are recorded in table 2.

**Table 2**

| volume fraction metal fibres | E (GPa) | k (Mpa.m^{½}) | H (GPa) | σ max (MPa) | ρ (%) |
|---|---|---|---|---|---|
| 10 | 126 | 3.7 | 3.9 | 96 | 99.7 |
| 20 | 135 | 7.0 | 3.4 | 175 | 99.2 |
| 30 | 142 | 7.4 | 3.3 | 224 | 99.8 |

The definitions of the measured properties and the comparative value of unfilled hydroxy apatite are recorded in example 1.

It appears from all the examples that the filled material has a lower hardness but a larger resistance to tear, larger tensile strength and larger modulus of elasticity than the ulfilled material. The density and the homogeneity of the resulting filled materials are high. Moreover it has been found that the metal fibres are not attacked or are hardly attacked during sintering when the sintering step is carried out in an inert atmosphere.

## Claims

1. A method of manufacturing a ceramic body filled with metal particles, characterized in that metal particles and ceramic particles are mixed wet in a hammer mill after which the mixed mass is dried and ground to a coarse powder, the powder is then compressed in a jig in the desired form after which the resulting intermediate product is densified by means of isostatic compression and is then sintered.

2. A method as claimed in Claim 1, characterized in that the coarse powder is provided in the jig in the form of thin layers, each layer being smoothed before the next layer is provided.

3. A method as claimed in Claim 1, characterized in that the coarse powder is compressed to the desired shape in the jig at a pressure from 10⁷ to 5.10⁷ Pa.

4. A method as claimed in Claim 1, characterized in that the densifying step is carried out by means of isostatic compression at a pressure of 2.10⁸ to 5.10⁸ Pa.

5. A method as claimed in Claim 1, characterized in that the sintering step is carried out in an inert atmosphere.

6. A method as claimed in Claim 1, characterized in that the sintering step is carried out at a temperature of 900 to 1000°C and a pressure of 3.10⁷ to 10⁸ Pa.

7. A method as claimed in Claim 1, characterized in that metal fibres having a length smaller than 2 mm and a diameter from 5 to 100 µm are used as metal particles.

8. A method as claimed in Claim 1, characterized in that fibres of a metal having a higher coefficient of linear expansion than that of the ceramic basic material are used as metal particles,

## Patentansprüche

1. Verfahren zum Herstellen eines mit Metallteilchen gefüllten keramischen Körpers, dadurch gekennzeichnet, daß Metallteilchen und Keramikteilchen in einer Schlagmühle naß vermischt werden, wonach die gemischte Masse getrocknet und zu einem grobkörnigen Pulver zermahlen wird, wonach das Pulver in einer Lehre zu der erwünschten Form gepreßt wird, wonach das auf diese weise gebildete Zwischenprodukt in einem isostatischen Preßvorgang verdichtet und danach gesintert wird.

2. Verfahren nach Anspruch 1, dadurch gekennzeichnet, daß das grobkörnige Pulver in Form dünner Schichten in der Lehre gegeben wird, wobei jede Schicht vor dem Anbringen der nächsten Schicht geglättet wird.

3. Verfahren nach Anspruch 1, dadurch gekennzeichnet, daß das grobkörnige Pulver in der Lehre vorzugsweise bei einem Druck von 10⁷ bis 5.10⁷ Pa zu der erwünschten Form gepreßt wird.

4. Verfahren nach Anspruch 1, dadurch gekennzeichnet, daß der Verdichtungsschritt durch einen isostatischen Preßvorgang bei einem Druck von 2.10⁸ bis 5.10⁸ Pa durchgeführt wird.

5. Verfahren nach Anspruch 1, dadurch gekennzeichnet, daß der Sintervorgang in einer inerten Atmosphäre durchgeführt wird.

6. Verfahren nach Anspruch 1, dadurch gekennzeichnet, daß der Sinterschritt vorzugsweise bei einer Temperatur von 900 bis 1000 °C und bei einem Druck von 3.10⁷ bis 10⁸ durchgeführt wird.

7. Verfahren nach Anspruch 1, dadurch gekennzeichnet, daß als Metallteilchen Metallfasern verwendet werden, deren Lange kleiner ist als 2 mm und deren Durchmesser 5 bis 100 µm beträgt.

8. Verfahren nach Anspruch 1, dadurch gekennzeichnet, daß als Metallteilchen Fasern aus einem Metall mit einem höheren Längsausdehungskoeffizienten als der des Ausgangsmaterials verwendet werden.

## Revendications

1. Procédé pour la réalisation d'un corps céramique rempli de particules métalliques, caractérisé en ce que les particules métalliques et les particules céramiques sont mélangées par voie humide dans un broyeur à marteaux, après quoi la masse mélangée est séchée et broyée de manière à obtenir une poudre grossière, la poudre est alors comprimée dans un moule de manière à présenter la forme désirée, le produit intermédiaire ainsi obtenu étant densifié par compression isostatique pour être fritté ensuite.

2. Procédé selon la revendication 1, caractérisé en ce que la poudre grossière soit formée dans le moule sous forme de couches minces, chaque couche étant aplanie avant que la couche suivante ne soit réalisée.

3. Procédé selon la revendication 1, caractérise en ce que la poudre grossière dans le moule est pressée dans la forme désirée à une pression comprise entre 10⁷ et 5.10⁷ Pa.

4. Procédé selon la revendication 1, caractérisé en ce que l'étape de densification par compression isostatique est effectuée à une pression comprise entre 2.10⁸ et 5.10⁸ Pa.

5. Procédé selon la revendication 1, caractérisé en ce que l'étape de frittage est effectuée dans une atmosphère inerte.

6. Procédé selon la revendication 1, caractérisé en ce que l'étape de frittage est effectuée à une température comprise entre 900 et 1000°C et à une pression comprise entre 3.10⁷ et 10⁸ Pa.

7. Procédé selon la revendication 1, caractérisé en ce que l'on utilise comme particules métalliques des fibres métalliques présentant une longueur inférieure à 2 mm et un diamètre compris entre 5 et 100 µm.

8. Procédé selon la revendication 1, caractérisé en ce que l'on utilise comme particules métalliques des fibres d'un métal présentant un coefficient de dilatation linéaire supérieur à celui du matériau de base céramique.
